# EUROPEAN PATENT SPECIFICATION

(11) **EP 3 717 032 B1**
(45) Date of publication and mention of the grant of the patent: **15.02.2023**
(21) Application number: 18822154.3
(22) Date of filing: 29.11.2018
(51) Int. Cl.: A61L 27/20, A61K 38/10, A61L 27/52, A61L 27/58, A61K 38/44, A61P 25/00

(54) **METHODS OF PREVENTING OR TREATING NEUROGENIC SHOCK**
VERFAHREN ZUR VORBEUGUNG ODER BEHANDLUNG VON NEUROGENEM SCHOCK
PROCÉDÉS DE TRAITEMENT PROPHYLACTIQUE OU THÉRAPEUTIQUE DE CHOC NEUROGÉNIQUE

(30) Priority: 29.11.2017 US 201762591860 P
(43) Date of publication of application: 07.10.2020
(73) Proprietor: Ramot at Tel-Aviv University Ltd., 6139201 Tel-Aviv (IL); The Medical Research, Infrastructure, And Health Services Fund Of The Tel Aviv Medical Center, 6423906 Tel-Aviv (IL)
(72) Inventor: ROCHKIND, Shimon, 6949903 Tel-Aviv (IL); NEVO, Zvi, 6139201 Tel-Aviv (IL)
(74) Representative: Durán-Corretjer, S.L.P.
(86) International application number: PCT/IL2018/051308
(87) International publication number: WO 2019/106671

(56) References cited:
- WO-A1-2009/022339
- WO-A1-2018/100511
- SHIMON ROCHKIND ET AL: "Recovery of Peripheral Nerve with Massive Loss Defect by Tissue Engineered Guiding Regenerative Gel", BIOMED RESEARCH INTERNATIONAL, vol. 2014, 1 January 2014 (2014-01-01), pages 1-7, XP055558667, ISSN: 2314-6133, DOI: 10.1155/2014/327578

## Description

### FIELD AND BACKGROUND OF THE INVENTION

The present invention, in some embodiments thereof, relates to methods of preventing or treating neurogenic shock.

Neurogenic shock, also known as vasogenic shock, is a devastating consequence of nerve injuries resulting from sudden loss of sympathetic tone which leads to autonomic instability manifested in e.g. hypotension, bradyarrhythmia, bradycardia, diaphragmatic breathing and temperature dysregulation. Neurogenic shock is most commonly a consequence of traumatic spinal cord injuries. Other causes of neurogenic shock that are far less common include spinal anesthesia, Guillain-Barre syndrome, autonomic nervous system toxins, transverse myelitis, and other neuropathies. Current treatment of neurogenic shock includes intravenous fluid resuscitation, vasopressors and inotropes.

Hydrated gels (hydrogels) are viscous, semisolid entities at physiological temperatures and pH which can be used for tissue engineering and regenerative medicine. For example, hyaluronic acid-based hydrogels provide a growth supportive milieu for cells and tissues such as for nerve regeneration (Suzuki et. al., 2003; Itoh et. al., 2005), while guiding migration and regeneration of nutritional-trophic and anti-oxidative agents.

International Patent Application Publication No. WO2009/022339 discloses the use of a hyaluronic acid-based hydrogel containing the anti-oxidant sodium dismutase and a laminin peptide for neural tissue regeneration and repair.

### SUMMARY OF THE INVENTION

According to an aspect of some embodiments of the present invention there is provided a composition for use in a method of treating neurogenic shock following nerve injury in a subject demonstrating at least one symptom of neurogenic shock, the method comprising implanting a composition comprising a hyaluronic acid, a laminin polypeptide and an antioxidant, at or near the nerve injury of the subject, thereby treating the neurogenic shock in the subject.

According to an aspect of some embodiments of the present invention there is provided a composition comprising a hyaluronic acid, a laminin polypeptide and an antioxidant, for use in treating neurogenic shock following nerve injury in a subject demonstrating at least one symptom of neurogenic shock.

According to an aspect of some embodiments of the present invention there is provided composition for use in a method of preventing or treating neurogenic shock following nerve injury in a subject in need thereof, the method comprising implanting within 48 hours following the nerve injury a composition comprising a hyaluronic acid, a laminin polypeptide and an antioxidant, at or near the nerve injury of the subject, thereby preventing or treating the neurogenic shock in the subject.

According to some embodiments of the invention, the implanting is effected within 24 hours following said nerve injury.

According to some embodiments of the invention, the implanting is effected within 12 hours following said nerve injury.

According to an aspect of some embodiments of the present invention there is provided a composition comprising a hyaluronic acid, a laminin polypeptide as set forth by SEQ ID NO: 1 and an antioxidant, for use in preventing or treating neurogenic shock following nerve injury in a subject, wherein the composition is implanted within 48 hours following said nerve injury.

According to some embodiments of the invention, the composition is implanted within 24 hours following the nerve injury.

According to some embodiments of the invention, the composition is implanted within 12 hours following the nerve injury.

According to some embodiments of the invention, the composition is implanted at or near the nerve injury of the subject.

According to some embodiments of the invention, the nerve injury is part of the central nervous system (CNS).

According to some embodiments of the invention, the nerve injury comprises spinal cord injury (SCI).

According to some embodiments of the invention, the nerve injury comprises traumatic brain injuries (TBI) or traumatic optic neuropathy (TON).

According to some embodiments of the invention, the subject is demonstrating at least one symptom of the neurogenic shock selected from the group consisting of instantaneous hypotension, warm flushed skin, priapism, peripheral vasodilatation, inability to get tachycardic, bradycardia, diaphragmatic breathing, respiratory arrest and organ dysfunction.

According to some embodiments of the invention, the subject is demonstrating at least one symptom of the neurogenic shock selected from the group consisting of hypotension, bradycardia and diaphragmatic breathing.

According to some embodiments of the invention, the antioxidant is superoxide dismutase (SOD).

According to some embodiments of the invention, the SOD comprises the amino acid sequence set forth by SEQ ID NO: 4.

According to some embodiments of the invention, the antioxidant is vitamin E.

According to some embodiments of the invention, the laminin polypeptide is set forth by SEQ ID NO: 1.

According to some embodiments of the invention, the antioxidant is superoxide dismutase (SOD) comprising the amino acid sequence set forth by SEQ ID NO: 4.

According to some embodiments of the invention, the hyaluronic acid, the antioxidant and the laminin polypeptide are cross linked.

According to some embodiments of the invention, the composition is formulated as a matrix.

According to some embodiments of the invention, the composition is formulated as a hydrogel.

According to some embodiments of the invention, the hyaluronic acid is provided at a concentration range of about 0.5-1.5 % in the hydrogel.

According to some embodiments of the invention, the laminin polypeptide is provided at a concentration range of about 20-100 µg/ml in the hydrogel.

According to some embodiments of the invention, the antioxidant is provided at a concentration range of about 5-40 µg/ml in the hydrogel.

According to some embodiments of the invention, the hyaluronic acid, the laminin polypeptide and the antioxidant are provided at a total concentration of about 0.01 - 0.6 % in the hydrogel.

According to some embodiments of the invention, the hyaluronic acid, the laminin polypeptide and the antioxidant are provided at a total concentration of about 0.4 % in the hydrogel.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

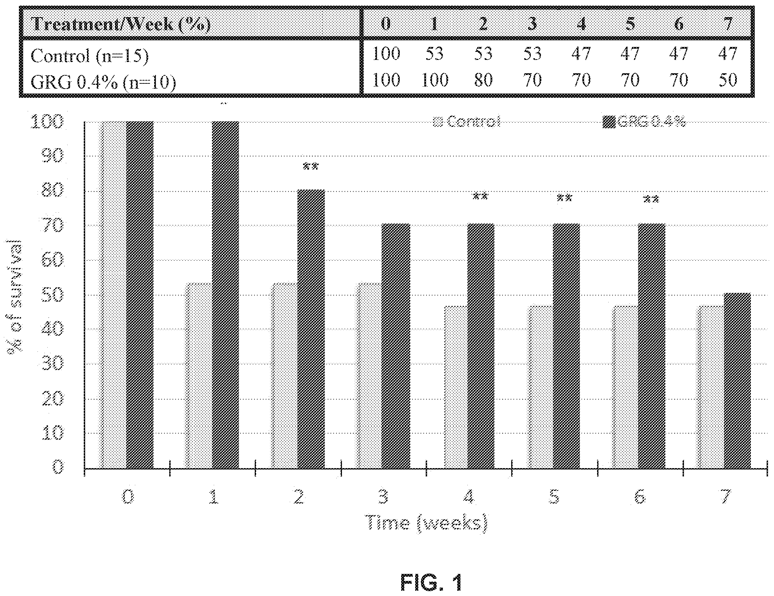

FIG. 1 shows a table and a graph summarizing survival rates (in percentages) of SCI rats implanted with guiding regenerative gel (GRG) as compared to control untreated SCI rats. * P < 0.05 versus control; ^{∗∗} P < 0.1 versus control.

### DESCRIPTION OF SPECIFIC EMBODIMENTS OF THE INVENTION

The present invention, in some embodiments thereof, relates to compositions for use in methods of preventing or treating neurogenic shock.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Neurogenic shock, also known as vasogenic shock, is a devastating consequence of nerve injuries resulting from sudden loss of sympathetic tone which leads to autonomic instability manifested in e.g. hypotension, bradyarrhythmia, and temperature dysregulation.

While reducing the invention to practice, the present inventors have now uncovered that a composition comprising hyaluronic acid, an anti-oxidant, a laminin peptide (SEQ ID NO: 1) can be used for prevention or treatment of neurogenic shock following nerve injury. When formulated as a gel the hydrogel is referred to herein as GRG for guiding regenerative gel.

As is illustrated hereinunder and in the Examples section, which follows, the present inventors demonstrate that implanting GRG at or near the site of spinal cord injury (SCI) had a protective effect against spinal shock and decreased the mortality rate in the SCI treated rats (Example 1, Figure 1).

Thus, according to a first aspect of the present invention, there is provided a method of treating neurogenic shock following nerve injury in a subject demonstrating at least one symptom of neurogenic shock, the method comprising implanting a composition comprising a hyaluronic acid, a laminin polypeptide and an antioxidant, at or near the nerve injury of the subject, thereby treating the neurogenic shock in the subject.

According to another aspect of the present invention, there is provided a composition comprising a hyaluronic acid, a laminin polypeptide as set forth by SEQ ID NO: 1 and an antioxidant, for use in treating neurogenic shock following nerve injury in a subject demonstrating at least one symptom of neurogenic shock.

According to another aspect of the present invention, there is provided a composition for use in a method of preventing or treating neurogenic shock following nerve injury in a subject in need thereof, the method comprising implanting within 48 hours following the nerve injury a composition comprising a hyaluronic acid, a laminin polypeptide and an antioxidant, at or near the nerve injury of the subject, thereby preventing or treating the neurogenic shock in the subject.

According to another aspect of the present invention, there is provided a composition comprising a hyaluronic acid, a laminin polypeptide as set forth by SEQ ID NO: 1 and an antioxidant, for use in preventing or treating neurogenic shock following nerve injury in a subject, wherein said composition is implanted within 48 hours following said nerve injury.

As used herein, the term "subject" refers to a mammalian subject (e.g., human being) of any gender and any age including neonatal, infant, juvenile, adolescent, adult and elderly adult.

According to specific embodiments of the invention, the term encompasses individuals who suffer from a pathology (i.e. neurogenic shock) as described below.

According to specific embodiment, the subject is demonstrating symptom(s) characterizing the pathology.

Thus, according to specific embodiments, the subject is demonstrating at least one symptom of neurogenic shock selected from the group consisting of instantaneous hypotension, warm flushed skin, priapism, peripheral vasodilatation, inability to get tachycardic, bradycardia, diaphragmatic breathing, respiratory arrest and organ dysfunction.

According to specific embodiments, the subject is demonstrating at least one symptom of neurogenic shock selected from the group consisting of hypotension, bradycardia, and diaphragmatic breathing.

Veterinary uses are also contemplated. Thus, according to specific embodiments, the components of the composition of the present invention are selected avoiding xeno responses.

The term "treating" refers to causing the reduction, remission, or regression of a pathology in a subject demonstrating symptom(s) characterizing the pathology. Those of skill in the art will understand that various methodologies and assays can be used to assess the reduction, remission or regression of a pathology.

As used herein, the term "preventing" refers to keeping a disease, disorder or condition from occurring in a subject who may be at risk for the disease, but does not yet display symptoms of the disease disorder or condition or has not yet been diagnosed as having the disease, disorder or condition. Those of skill in the art will understand that various methodologies and assays can be used to assess the development of a pathology.

As used herein the phrase "nerve injury" refers to any disorder, disease, or condition exhibiting damage (*i.e.*, non-functioning tissue, cancerous or pre-cancerous tissue, broken tissue, fractured tissue, fibrotic tissue, or ischemic tissue) or loss (e.g., following a trauma, an infectious disease, a genetic disease, and the like) of neuronal tissue which may lead to neurogenic shock. Non-limiting examples of nerve injuries include spinal cord injury (SCI), cervical cord trauma, or high thoracic cord injuries, head trauma, traumatic brain injury (TBI), traumatic optic neuropathy (TON), Guillain-Barre syndrome, autonomic nervous system toxins, transverse myelitis

According to specific embodiments the nerve injury is caused by trauma and not by a disease.

According to specific embodiments the nerve injury is part of the central nervous system (CNS).

The term "central nervous system (CNS)", as used herein can refer to a subject's brain, spinal cord and/or optic nerve.

According to specific embodiments, the nerve injury comprises spinal cord injury.

As used herein, the phrase "spinal cord injury (SCI)" refers to an injury to the spinal cord that is caused by trauma and not by disease. Spinal cord injuries have many causes; according to specific embodiments, the SCI is caused by a major trauma from motor vehicle accidents, falls, sports injuries or violence. Depending on where the spinal cord and nerve roots are damaged, the symptoms can vary widely e.g. from pain to paralysis to incontinence. The SCIs can be incomplete or complete injury which means a total loss of function. According to specific embodiments, the SCI is complete SCI.

According to specific embodiments, the nerve injury comprises traumatic brain injury (TBI).

As used herein, the phrase "traumatic brain injury (TBI)" refers to brain injury caused by trauma and not by disease. TBIs have many causes; according to specific embodiment, the TBI is caused by falls, vehicle collisions, sports collisions or combats. The phrase includes both mild and severe TBI including closed-head injuries, concussions or contusions and penetrating head injuries.

According to specific embodiments, the nerve injury comprises traumatic optic neuropathy (TON).

As used herein, the phrase "traumatic optic neuropathy (TON)" refers to injury to the optic nerve caused by trauma and not by disease. According to specific embodiments, TON results in vision loss, which may be partial or complete. TONs have many causes; according to specific embodiments, the TON is caused by an anatomical disruption of the optic nerve fibers from penetrating orbital trauma, bone fragments within the optic canal or nerve sheath hematomas.

As used herein, the phrase "neurogenic shock" refers to a shock resulting in low blood pressure, occasionally with a slowed heart rate that is attributed to the disruption of the autonomic pathways that occurs following nerve injury. Methods of diagnosing neurogenic shock and evaluating its progression are known in the art and include, but not limited to, radiographic imaging, hemodynamic monitoring and/or clinical exam. Symptoms of neurogenic shock include, but are not limited to instantaneous hypotension due to sudden, massive vasodilation, warm, flushed skin due to vasodilation and inability to vasoconstrict, priapism, also due to vasodilation; inability to get tachycardic, bradycardia, diaphragmatic breathing due to loss of nervous control of the intercostal muscles (typically due to an injury below the 5th cervical vertebra), respiratory arrest immediately following the injury due to loss of nervous control of the diaphragm (typically due to an injury above the 3rd cervical vertebra), organ dysfunction or death.

According to specific embodiments, the methods and the compositions of the present invention increase the survival of the subject.

According to specific embodiments the increase in survival is at least 1.5 fold, at least 2 fold, at least 3 fold, at least 5 fold, at least 10 fold, or at least 20 fold as compared to same in the absence of implanting the composition of the present invention which may be obtained from databases and the known literature.

According to other specific embodiments the increase in survival is by at least 5 %, by at least a 10 %, at least 20 %, at least 30 %, at least 40 %, at least 50 %, at least 60 %, at least 70 %, at least 80 %, at least 90 %, at least 95 % or at least 100 % as compared to same in the absence of implanting the composition of the present invention which may be obtained from databases and the known literature.

The compositions of the present invention comprise a hyaluronic acid, a laminin polypeptide and an antioxidant.

As used herein, the term "hyaluronic acid (HA)", also known as hyaluronan, hyaluronate, refers to an unsulphated glycosaminoglycan composed of repeating disaccharide units of N-acetylglucosamine (GlcNAc) and glucuronic acid (GlcUA) linked together by alternating beta- 1, 4 and beta- 1, 3 glycosidic bonds. According to specific embodiments the hyaluronic acid is Na-HA. According to specific embodiments, the hyaluronic acid has a molecular weight from about 10⁴ Daltons to about 3 x 10⁶ Daltons. The molecular weight of HA can be evaluated by e.g. viscosity measurement with a digital viscosimeter Brookfield brand Cone/Plate DVII + Per (Brookfield Engineering Laboratories Inc. Middleboro, MA 02346-1031 USA). The molecular weight of HA can be calculated as well by the discrepancy between the figure obtained in Dische's assays versus the data obtained by Park-Johnson (Park J.T. Johnson M.J. A submicrodetermination of glucose J. Biol. Chem. 181, 149-151, 1949) determination for reducing sugars.

The hyaluronic acid described herein includes naturally occurring HA synthetic HA or a combination of same. According to specific embodiments, the hyaluronic acid can be extracted and isolated from an organism such as rooster combs or umbilical cords or from bacterial cultures such as those of hemolytic group A or C Streptococci, or can be synthetically produced using methods which are well known in the art.

According to specific embodiments of the invention, the hyaluronic acid is pure enough from chemical or biological constituents so that it is biologically inert having a low rate of reactivity with other substances under ordinary conditions.

According to specific embodiments of the invention, the hyaluronic acid is pure enough so that it is biocompatible, e.g., when in contact with cells, tissues or body fluid of an organism does not induce adverse effects such as immunological reactions and/or rejections, cellular death, and the like.

According to specific embodiments, the hyaluronic acid is at least 80 %, at least 90 %, at least 95 %, at least 98 % or at least 99 % pure.

According to specific embodiments, the hyaluronic acid is analytical (i.e. 99.5 % - 100 %) or pharmaceutical grade (98 % - 100 % hyaluronic acid).

The hyaluronic acid described herein is capable of forming highly hydrated gels in aqueous solutions.

Total content of HA in the composition of the present invention can be determined by methods known in the art such as, but not limited to the content of uronic acids (lucuronic acid) by the routine test of Dische (Dische Z. A new specific color reaction of hexuronic Acids. J. Biol. Chem, 167, 189-197, 1947) employing the carbazol reagent.

As used herein the term "laminin" refers to the family of extracellular matrix glycoproteins, which form the major noncollagenous constituent of basement membrane. Laminins have been implicated in a wide variety of biological processes including cell adhesion, differentiation, migration, signaling, neurite outgrowth and metastasis. Laminins are composed of 3 non identical chains: laminin alpha, beta and gamma, each encoded by a distinct gene.

As used herein the phrase "laminin polypeptide" refers to an amino acid sequence which comprises at least 4 consecutive amino acids of a laminin polypeptide and which exhibits a biological activity (e.g., support cell survival, growth, proliferation, differentiation and/or migration).

According to some embodiments of the invention the laminin polypeptide can include an amino acid sequence of a laminin alpha-chain such as LAMA1 (e.g., GenBank Accession No. NP_005550.2), LAMA2 (e.g., GenBank Accession Nos. NP_000417.2 and NP_001073291.1), LAMA3 (e.g., GenBank Accession Nos. NP_937762.1 and NP_000218.2), LAMA4 (e.g., GenBank Accession Nos. NP_001098677.1, NP_001098676.1, NP_002281.2, NP_001098679.1, and NP_001098678.1), and LAMA5 (e.g., GenBank Accession No. NP_005551.3); a laminin beta-chain such as LAMB1 (e.g., GenBank Accession No. NP_002282.1), LAMB2 (e.g., GenBank Accession No. NP_002283.3), LAMB3 (e.g., GenBank Accession Nos. NP_000219.2 and NP_001017402.1) and LAMB4 (e.g., GenBank Accession No. NP_031382.2); and/or a laminin gamma-chain such as LAMC1 (e.g., GenBank Accession No. NP_002284.3), LAMC2 (e.g., GenBank Accession Nos. NP_005553.2 and NP_061486.2) and LAMC3 (e.g., GenBank Accession No. NP_006050.3).

According to specific embodiments of the invention, the laminin polypeptide includes a repeated amino acid sequence (e.g., a 4 or 5 amino acid repeated sequence) of a laminin sequence.

Non-limiting examples of laminin polypeptides which can be included in the composition of the invention include the peptides set forth in SEQ ID NOs: 1, 2 or 3. [KSIKVAVRSYIGSRCV (SEQ ID NO: 1), IKVAV (SEQ ID NO:2), YIGSR (SEQ ID NO:3)].

According to the invention, the laminin polypeptide is set forth by SEQ ID NO: 1 (KSIKVAVRSYIGSRCV).

According to specific embodiments the laminin polypeptide is 5-50, 5-25, 5-20, 16-50 or 16-25 amino acids long.

According to specific embodiments, the laminin polypeptide is at least 16 amino acids long but shorter than 50 amino acids long.

The terms "polypeptide" or "peptide" which are interchangeably used herein, encompass native peptides (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992), which is incorporated by reference as if fully set forth herein. Further details in this respect are provided hereinunder.

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted for synthetic non-natural acid such as TIC, naphthylelanine (Nol), ring-methylated derivatives of Phe, halogenated derivatives of Phe or o-methyl-Tyr.

In addition to the above, the peptides of the present invention may also include one or more modified amino acids or one or more non-amino acid monomers (e.g. fatty acids, complex carbohydrates etc).

The term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Tables 1 and 2 below list naturally occurring amino acids (Table 1) and non-conventional or modified amino acids (e.g., synthetic, Table 2) which can be used with the present invention.

**Table 1**

| ***Amino Acid*** | ***Three-Letter Abbreviation*** | ***One-letter Symbol*** |
|---|---|---|
| alanine | Ala | A |
| Arginine | Arg | R |
| Asparagine | Asn | N |
| Aspartic acid | Asp | D |
| Cysteine | Cys | C |
| Glutamine | Gln | Q |
| Glutamic Acid | Glu | E |
| glycine | Gly | G |
| Histidine | His | H |
| isoleucine | Iie | I |
| leucine | Leu | L |
| Lysine | Lys | K |
| Methionine | Met | M |
| phenylalanine | Phe | F |
| Proline | Pro | P |
| Serine | Ser | S |
| Threonine | Thr | T |
| tryptophan | Trp | W |
| tyrosine | Tyr | Y |
| Valine | Val | V |
| Any amino acid as above | Xaa | X |

**Table 2**

| ***Non-conventional amino acid*** | ***Code*** | ***Non-conventional amino acid*** | ***Code*** |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbonyl- | Norb | L-N-methylglutamine | Nmgin |
| carboxylate | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcycloheaylalanine | Mchexa |
| D-α-methylarginine | Dmarg | α-methylcyclopentylalanine | Mcpen |
| D-oC-methyla sparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3 -aminopropyl)glycine | Norn |
| D-α-methylisoleucine | Dmile | N- amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmom | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cyclododeclglycine | Ncdod |
| D-α-methylalnine | Dnmala | N-cyclooctylglycine | Ncoct |
| D-α-methylarginine | Dnmarg | N-cyclopropylglycine | Ncpro |
| D-oC-methyla sparagine | Dnmasn | N-cycloundecylglycine | Ncund |
| D-α-methylasparatate | Dnmasp | N-(2,2-diphenylethyl)glycine | Nbhm |
| D-α-methylcysteine | Dnmcys | N-(3,3-diphenylpropyl)glycine | Nbhe |
| D-N-methylleucine | Dnmleu | N-(3-indolylyethyl) glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylomithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methyethyl)glycine | Nva |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-oC-methylarginine | Marg | L-oC-methylasparagine | Masn |
| L-α-methylaspartate | Masp | L-α-methyl-*t*-butylglycine | Mtbug |
| L-α-methylcysteine | Mcys | L-methylethylglycine | Metg |
| L-oC-methylglutamine | Mgln | L-oC-methylglutamate | Mglu |
| L-α-methylhistidine | Mhis | L-α-methylhomo phenylalanine | Mhphe |
| L-α-methylisoleucine | Mile | N-(2-methylthioethyl)glycine | Nmet |
| D-N-methylglutamine | Dnmgln | N-(3 -guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl)glycine | Nser |
| D-N-methylisoleucine | Dnmile | N-(imidazolylethyl)glycine | Nhis |
| D-N-methylleucine | Dnmleu | N-(3 -indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-oC-methylarginine | Marg | L-oC-methylasparagine | Masn |
| L-α-methylaspartate | Masp | L-α-methyl-*t*-butylglycine | Mtbug |
| L-α-methylcysteine | Mcys | L-methylethylglycine | Metg |
| L-oC-methylglutamine | Mgln | L-oC-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | mser | L-α,-methylthreonine | Mthr |
| L-α-methylvaline | Mtrp | L-α-methyltyrosine | Mtyr |
| L-α-methylleucine | Mval Nnbhm | L-N-methylhomophenylalanine | Nmhphe |
| N-(N-(2,2-diphenylethyl) | | N-(N-(3,3-diphenylpropyl) | |
| carbamylmethyl-glycine | Nnbhm | carbamylmethyl(1)glycine | Nnbhe |
| 1-carboxy-1-(2,2-diphenyl ethylamino)cyclopropane | Nmbc | | |

The peptides of the present invention are preferably utilized in a linear form, although it will be appreciated that in cases where cyclicization does not severely interfere with peptide characteristics, cyclic forms of the peptide can also be utilized.

Since the present peptides can be utilized in therapeutics or diagnostics which require the peptides to be in soluble form, the peptides of the present invention preferably include one or more non-natural or natural polar amino acids, including but not limited to serine and threonine which are capable of increasing peptide solubility due to their hydroxyl-containing side chain.

The peptides of the present invention may be synthesized by any techniques that are known to those skilled in the art of peptide synthesis. For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Academic Press (New York), 1965.

In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid can then either be attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final peptide compound. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide and so forth. Further description of peptide synthesis is disclosed in U.S. Pat. No. 6,472,505.

A preferred method of preparing the peptide compounds of the present invention involves solid phase peptide synthesis.

Large scale peptide synthesis is described by Andersson Biopolymers 2000;55(3):227-50.

In cases where large amounts or long polypeptides (e.g., longer than 20 amino acids) are desired, the polypeptides of the present invention can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680, Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

The composition further comprises an antioxidant which can protect cells or macromolecules (e.g., the polysaccharide) from oxidative stress (oxidative damage caused by free radicals). Thus, the antioxidant can extend the survival of the macromolecules by preventing their oxidative depolymerization.

Non-limiting examples of suitable antioxidants include molecules such as glutathione, vitamin C (sodium ascorbate), vitamin E (tocopherols and tocotrienols), N-Ac-L-cysteine, hydroquinone, glutamate, or enzymes such as catalase, superoxide dismutase, glutathione peroxidase or other peroxidases, and glucose-6-phosphate dehydrogenase (G6PD) (see Osmen I., Naziroglu M., Okutan R. Comparative study of antioxidant enzymes in tissues surrounding implant in rabbits. Cell. Biochem. Funct. 24:275-281, 2006).

According to specific embodiment, the antioxidant is vitamin E.

According to specific embodiments, the antioxidant is superoxide dismutase (SOD).

As used herein, the term "superoxide dismutase" E.C. No: 1.15.1.1 refers to an enzyme that alternately catalyzes the dismutation (or partitioning) of the superoxide (O₂⁻) radical into either ordinary molecular oxygen (O₂) or hydrogen peroxide (H₂O₂). Superoxide dismutase, in addition to its known activity as an antioxidant, can also serve as an anti-inflammatory agent when used *in vivo.* Non-limiting examples of superoxide dismutase (SOD) enzymes which can be used in the composition of the invention include, SOD-1 (soluble), SOD-2 (mitochondrial) or SOD-3 (extracellular), such as homo sapiens soluble superoxide dismutase 1 (SOD-1) GenBank Accession No. NP_000445 (SEQ ID NO: 4); homo sapiens mitochondrial superoxide dismutase 2 (SOD-2) GenBank Accession Nos. NP_001019637.1 (isoform B), NP_001019636.1 (isoform A), NP_000627.2 (isoform A); homo sapiens extracellular superoxide dismutase 3 (SOD-3) GenBank Accession No. NP_003093.2; Saccharomyces cerevisiae SOD-1 GenBank Accession No. NP_012638.1; and Rattus norvegicus SOD-1 GenBank Accession No. NP_058746.

According to specific embodiments, the SOD comprises the amino acid sequence set forth by SEQ ID NO: 4.

The antioxidant of the invention can be produced by recombinant techniques, e.g. as described in Hartman JR., et al., 1986 (Proc. Natl. Acad. Sci. USA, Vol: 83, pp 7142-7146). For example, a polynucleotide encoding superoxide dismutase 1 (GenBank Accession No. NM_000454; SEQ ID NO: 5) can be ligated into a nucleic acid construct suitable for expression in a host cell (e.g., bacterial cell, yeast cell, mammalian cell). Such a nucleic acid construct includes a promoter sequence for directing transcription of the polynucleotide sequence in the cell in a constitutive or inducible manner, and may also include sequences which render this vector suitable for replication and integration in prokaryotes, eukaryotes, or preferably both (e.g., shuttle vectors); transcription and translation initiation sequence, enhancers, transcription and translation terminator, and a polyadenylation signal which may increase the efficiency of mRNA translation; a signal sequence for secretion; sequences engineered to enhance stability, production, purification, yield or toxicity of the expressed polypeptide.

The antioxidant can be recovered and purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromate-focusing and differential solubilization.

According to specific embodiments of the invention, the antioxidant is retrieved so that it is pure enough from chemical or biological constituents to allow for the effective use of the recombinant polypeptide as an antioxidant. Activity of the anti-oxidant, e.g. SOD, may be determined by methods well known in the art and include measurement at 560 nm as the rate of suppression of reduction of nitrotetrazolium blue when superoxide anion radical was generated during oxidation of xanthine by xanthine oxidase.

According to specific embodiments, the antioxidant (e.g. SOD) is at least 80 %, at least 90 %, at least 95 %, at least 98 % or at least 99 % pure.

According to specific embodiments, the antioxidant (e.g. SOD) is analytical or pharmaceutical grade anti-oxidant.

According to specific embodiments, the components of the composition are cross-linked.

According to specific embodiments, the hyaluronic acid, the antioxidant and the laminin peptide of the composition are cross-linked.

Cross-linking (*i.e*., binding via covalent or ionic bonds) of the components comprised in the composition can be performed using any cross-linking or coupling agent known in the art. Basically the principles of cross linking are combining free primary amino groups with carboxyl groups, or oxidizing in between close hydroxyl groups, forming reactive aldehydes, to interact either among themselves or with amines of additional conjugate may be formed via tiol residues.

According to specific embodiments, cross-linking does not affect the biological activities of the bonded elements.

Non-limiting examples of suitable cross-linking agents include dimethyl suberimidate (an imidoester cross linker); Bis(Sulfosuccinimidyl) suberate (BS3; an NHS-ester cross linker); formaldehyde; 1-Ethyl-3-[3-dimethylaminopropyl]carbodiimide hydrochloride (EDC; the carbodiimide cross linker); N-hydroxyuccinimide (NHS) [Mao J.S, et al., Biomaterials. 24,1621-1629, 2003; Choi Y.S., et al., J. Biomed. Mater. Res. 48,631-639, 1999; Richert L., et al., Biomacromolecules, 5, 284-294, 2004)]; Divinyl sulfone (DVS); and genipin [Sung H.W., et al., J Biomed. Mater. Res. A, 64A:427-438, 2003; Chen SC., et al., J. Control Release. 96, 285-300, 2004; Mwale F., et al., Tissue Eng., 11, 130-40, 2005; Chen H., et al., Biomacromolecules, 7, 2091-2098, 2006]. For *ex vivo* or *in vivo* cross-linking photo-reactive amino acid analogs (e.g., diazirine analogs to leucine and methionine) can be added to the composition and following exposure to ultraviolet light, the diazirines are activated and bind to interacting side chains (e.g., carboxyl or amino groups).

According to specific embodiments of the invention, cross-linking is performed using a non-toxic and/or biocompatible agent. Examples include, but are not limited to 3-dimenthy-aminoprophyl)-N-ethyl carbodiimide (EDC-N; Sigma-Aldrich- Fluka, St Louis, Missouri 63178, Catalogue No. 03459), divinyl sulfone (DVS; Sigma, Catalogue No. V-370-0) and genipin (Sigma Catalogue No. G-4796).

According to specific embodiments, the composition described herein can increase neuronal cells survival, support neuronal regeneration and/or prevent formation of a glial scar.

According to specific embodiments, the composition is generated according to the teachings of International Patent Application Publication No. WO2009/022339, the contents of which are incorporated herein in their entirety.

It should be noted that since the components comprised in the composition of the invention can be prepared using synthetic or recombinant techniques they are obtainable sterile preparations of analytical or pharmaceutical grade.

According to specific embodiments, the composition is formulated as a hydrogel.

As used herein, the term "hydrogel" refers to a material comprising the composition of some embodiment of the invention and water, in which the water constitutes more than 40 %.

According to specific embodiments of the invention, the hydrogel comprises at least about 50 %, at least about 60 % water, at least about 70 % water, at least about 80 % water, at least about 90 % water, at least about 95 % water, at least about 96 % water, at least about 97 % water, at least about 98 % water, at least about 99 % water.

According to specific embodiments, the hydrogel is viscous (e.g. approximately 0cP during no movement and 110-130cP during movement).

According to specific embodiments, the hydrogel is transparent.

According to specific embodiments, the hyaluronic acid is provided at a concentration range of about 0.3-2 %, e.g., about 0.4-1.8 %, e.g., about 0.5-1.6, e.g., about 0.5-1.5 %, e.g., about 0.6-1.4 %, e.g., about 0.8-1.2 %, e.g., about 1.2 % in the composition e.g. hydrogel.

According to a specific embodiment, the hyaluronic acid is provided at a concentration range of about 0.5-1.5 % in the composition e.g. hydrogel.

According to some embodiments, the laminin polypeptide (e.g., SEQ ID NO: 1) is provided at a concentration range of about 10-200 µg/ml, e.g., about 20-100 µg/ml, e.g., about 50 µg/ml in the composition e.g. hydrogel.

According to a specific embodiment, the laminin polypeptide is provided at a concentration range of about 20-100 µg/ml in the composition e.g. hydrogel.

According to some embodiments of the invention, the antioxidant (e.g. superoxide dismutase) is provided at a concentration range of about 8 µM (about 0.25 microgram/ml) to 8 mM (about 250 microgram/ml) in the hydrogel. For example, the antioxidant (e.g. superoxide dismutase) can be provided at a concentration range of about 0.5 µg/ml to about 200 µg/ml, e.g., from about 1 µg/ml to about 100 µg/ml, e.g., from about 2 µg/ml to about 80 µg/ml, e.g., from about 4 µg/ml to about 40 µg/ml, e.g., from about 5 µg/ml to about 50 µg/ml, e.g., from about 10 µg/ml to about 50 µg/ml, e.g., from about 15 µg/ml to about 40 µg/ml, e.g., from about 20 µg/ml to about 30 µg/ml, e.g., about 25 µ/ml.

According to specific embodiments, the antioxidant is provided at a concentration range of about 5-40 µg/ml in the composition e.g. hydrogel.

According to specific embodiments, the ratio between the hyaluronic acid, the laminin polypeptide and the antioxidant in the composition e.g. hydrogel is between HA 0.01 mg: laminin polypeptide 50 µg: SOD 250 µg per ml to HA 1.2 mg: laminin polypeptide 50 µg: SOD 250 µg per ml.

According to a specific embodiment, the ratio between the hyaluronic acid, the laminin polypeptide and the antioxidant in the composition e.g. hydrogel is approximately HA 0.4mg: laminin polypeptide 50 µg: SOD 250 µg per ml.

According to specific embodiments, the hyaluronic acid, the laminin polypeptide and the antioxidant are provided at a total concentration of about 0.01 - 0.6 %.

According to specific embodiments, the hyaluronic acid, the laminin polypeptide and the antioxidant are provided at a total concentration of about 0.02 - 0.5 %.

According to specific embodiments, the hyaluronic acid, the laminin polypeptide and the antioxidant are provided at a total concentration of about 0.4 %.

According to specific embodiments, the hyaluronic acid, the laminin polypeptide and the antioxidant are provided at a total concentration of about 0.02 %.

According to specific embodiments of the invention, the hydrogel is lyophilized by methods well known in the art such that a dry matrix is obtained.

According to specific embodiments, the dry mix comprises less than 50 %, less than 30 %, less than 10 %, less than 5 %, less than 2 %, less than 1 %, or less than 0.5 % water. It should be noted that water-free matrices can be preserved for long periods of time without being subjected to enzymatic degradation or contamination (e.g., by microorganisms).

Thus, according to specific embodiments, the composition is formulated as a matrix.

As used herein the phrase "matrix" refers to a two-dimensional or a three-dimensional scaffold (also referred to herein as supporting framework) comprising the composition of the invention. The scaffold may further provide mechanical stability and support.

The matrix can be kept in a dry or wet form, or can be frozen according to the intended use.

According to specific embodiments, the dry matrix can be further hydrated in an aqueous solution (e.g., water) until a hydrogel is formed.

According to specific embodiments, the dimensions of the matrix vary according with the nerve injury. For example, the size of the matrix can be smaller than or substantially the same size as the nerve injury. Alternatively, the size of the matrix can be larger than the nerve injury.

Scaffold material may comprise natural or synthetic organic polymers that can be gelled, or polymerized or solidified (e.g., by aggregation, coagulation, hydrophobic interactions, or cross-linking) into a two-dimensional or a three-dimensional structure.

The scaffold of the present invention may be made uniformly of a single polymer, co-polymer or blend thereof. However, it is also possible to form a scaffold according to the invention of a plurality of different polymers. There are no particular limitations to the number or arrangement of polymers used in forming the scaffold. Any combination which is biocompatible, may be formed into fibers, and degrades at a suitable rate, may be used.

Both the choice of polymer and the ratio of polymers in a co-polymer may be adjusted to optimize the stiffness of the scaffold. The molecular weight and cross-link density of the scaffold may also be regulated to control both the mechanical properties of the scaffold and the degradation rate (for degradable scaffolds). The mechanical properties may also be optimized to mimic those of the tissue at the implant site.

Polymers used in scaffold material compositions may be biocompatible, biodegradable and/or bioerodible and may act as adhesive substrates for cells. In exemplary embodiments, structural scaffold materials are easy to process into complex shapes and have a rigidity and mechanical strength suitable to maintain the desired shape under in vivo conditions.

In certain embodiments, the structural scaffold materials may be non-resorbing or non-biodegradable polymers or materials. Such non-resorbing scaffold materials may be used to fabricate materials which are designed for long term or permanent implantation into a host organism.

The phrase "non-biodegradable polymer", as used herein, refers to a polymer or polymers which at least substantially (i.e. more than 50 %) do not degrade or erode in vivo. The terms "non-biodegradable" and "non-resorbing" are equivalent and are used interchangeably herein. Examples of biocompatible non-biodegradable polymers which are useful as scaffold materials include, but are not limited to, polyethylenes, polyvinyl chlorides, polyamides such as nylons, polyesters, rayons, polypropylenes, polyacrylonitriles, acrylics, polyisoprenes, polybutadienes and polybutadiene-polyisoprene copolymers, neoprenes and nitrile rubbers, polyisobutylenes, olefinic rubbers such as ethylene-propylene rubbers, ethylene-propylene-diene monomer rubbers, and polyurethane elastomers, silicone rubbers, fluoroelastomers and fluorosilicone rubbers, homopolymers and copolymers of vinyl acetates such as ethylene vinyl acetate copolymer, homopolymers and copolymers of acrylates such as polymethylmethacrylate, polyethylmethacrylate, polymethacrylate, ethylene glycol dimethacrylate, ethylene dimethacrylate and hydroxymethyl methacrylate, polyvinylpyrrolidones, polyacrylonitrile butadienes, polycarbonates, polyamides, fluoropolymers such as polytetrafluoroethylene and polyvinyl fluoride, polystyrenes, homopolymers and copolymers of styrene acrylonitrile, cellulose acetates, homopolymers and copolymers of acrylonitrile butadiene styrene, polymethylpentenes, polysulfones, polyesters, polyimides, polyisobutylenes, polymethylstyrenes, and other similar compounds known to those skilled in the art.

In other embodiments, the structural scaffold materials may be a "bioerodible" or "biodegradable" polymer or material.

The phrase "biodegradable polymer" as used herein, refers to a polymer or polymers which degrade in vivo, and wherein erosion of the polymer or polymers over time occurs concurrent with or subsequent to release of the composition. The terms "biodegradable" and "bioerodible" are equivalent and are used interchangeably herein.

Such bioerodible or biodegradable scaffold materials may be used to fabricate temporary structures. Examples of biocompatible biodegradable polymers which are useful as scaffold materials include, but are not limited to, polylactic acid, polyglycolic acid, polycaprolactone, and copolymers thereof, polyesters such as polyglycolides, polyanhydrides, polyacrylates, polyalkyl cyanoacrylates such as n-butyl cyanoacrylate and isopropyl cyanoacrylate, polyacrylamides, polyorthoesters, polyphosphazenes, polypeptides, polyurethanes, polystyrenes, polystyrene sulfonic acid, polystyrene carboxylic acid, polyalkylene oxides, alginates, agaroses, dextrins, dextrans, polyanhydrides, biopolymers such as collagens and elastin, alginates, chitosans, glycosaminoglycans, and mixtures of such polymers. In still other embodiments, a mixture of non-biodegradable and bioerodible and/or biodegradable scaffold materials may be used to form a biomimetic structure of which part is permanent and part is temporary.

According to specific embodiments, PLA, PGA and PLA/PGA copolymers are used for forming the scaffolds of the present invention.

In an exemplary embodiment, scaffolds materials may comprise naturally occurring substances, such as, fibrinogen, fibrin, thrombin, chitosan, collagen, alginate, poly(N-isopropylacrylamide), albumin, collagen, synthetic polyamino acids, prolamines, polysaccharides such as alginate, heparin, and other naturally occurring biodegradable polymers of sugar units.

According to specific embodiments, the scaffolds of the present invention are porous. The porosity may be controlled by a variety of techniques known to those skilled in the art.

According to a specific embodiment of the present invention, the scaffolds are fabricated from synthetic biomaterials and are capable of conducting electricity and naturally eroding inside the body. In an exemplary embodiment, the scaffolds comprise a biocompatible polymer capable of conducting electricity e.g. a polypyrrole polymer. Polyaniline, polyacetyline, poly-p-phenylene, poly-p-phenylene-vinylene, polythiophene, and hemosin are examples of other biocompatible polymers that are capable of conducting electricity and may be used in conjunction with the present invention. Other erodible, conducting polymers are well known (for example, see Zelikin et al., Erodible Conducting Polymers for Potential Biomedical Applications, Angew. Chem. Int. Ed. Engl., 2002, 41(1): 141-144). Any of the foregoing electrical conducting polymers can be applied or coated onto a malleable or moldable scaffold.

The scaffolds may be made by any of a variety of techniques known to those skilled in the art. Salt-leaching, porogens, solid-liquid phase separation (sometimes termed freeze-drying), and phase inversion fabrication may all be used to produce porous scaffolds. Fiber pulling and weaving (see, e.g. Vacanti, et al., (1988) Journal of Pediatric Surgery, 23: 3-9) may be used to produce scaffolds having more aligned polymer threads. Those skilled in the art will recognize that standard polymer processing techniques may be exploited to create polymer scaffolds having a variety of porosities and microstructures.

Scaffold materials are readily available to one of ordinary skill in the art, usually in the form of a solution (suppliers are, for example, BDH, United Kingdom, and Pronova Biomedical Technology a.s. Norway). For a general overview of the selection and preparation of scaffolding materials, see the American National Standards Institute publication No. F2064-00 entitled Standard Guide for Characterization and Testing of Alginates as Starting Materials Intended for Use in Biomedical and Tissue Engineering Medical Products Applications".

According to specific embodiments the scaffold comprises a biodegradable membrane e.g. a dura film such as Lyodura, AESCLTLAP.

According to specific embodiments of the invention, the composition further comprises ex-vivo seeded cells such as stem cells or differentiated cells (e.g. neuronal progenitor cells).

Non-limiting examples of stem cells which can be used by the invention include embryonic stem cells, induced pluripotent stem cells (iPS), neuronal progenitor cells, hematopoietic stem cells (e.g., bone marrow stem cells, cord blood cells, peripheral blood stem cells), adult stem cells and mesenchymal stem cells.

According to some embodiments of the invention, the stem cells are neuronal progenitor cells (such as those obtained from embryonic or fetal neuronal tissue or brain).

According to specific embodiments, the differentiated cells are neural cells.

According to other specific embodiments, of the invention, the composition does not comprise ex-vivo seeded cells such as stem cells or differentiated cells (e.g. neuronal progenitor cells).

Those of skills in the art are capable of determining when and how to implant the composition within the subject. See for example, Artzi Z, et al., 2005, J. Clin. Periodontol. 32: 193-9; Butler CE and Prieto VG, 2004, Plast. Reconstr. Surg. 114: 464-73.

According to specific embodiments, the composition of the present invention is implanted during a surgical procedure effected for treating the nerve injury in the subject.

According to specific embodiments, the composition of the present invention is implanted locally at or near the site of the nerve injury.

For example, for treating spinal cord injuries, the composition is implanted directly into the lesion (e.g. into the epicenter of the injury) and near the lesion (e.g. at distance of approximately 0.5 cm from the injured site.). Following implantation the implants can be fixed by surgical adhesives (e.g. BioGlue, CryoLife); and finally the muscular and cutaneous planes are closed and sutured.

According to specific embodiments, the composition is implanted within 12 hours, within 24 hours, within 48 hours, within 72 hours or within 96 hours following the nerve injury, each possibility represents a separate embodiment of the present invention.

According to specific embodiments, the composition is implanted within 48 hours following the nerve injury.

According to specific embodiments, the composition is implanted within 24 hours following the nerve injury.

According to specific embodiments, the composition is implanted within 12 hours following the nerve injury.

The compositions of the invention may, if desired, be presented in a pack or dispenser device, such as an FDA approved kit, or an article of manufacture (with packaging material), which may contain one or more unit dosage forms containing the active ingredient. The pack may, for example, comprise metal or plastic foil, such as a blister pack. The pack or dispenser device may be accompanied by instructions for administration, implantation and/or treating a subject. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. The compositions of the invention formulated in a compatible pharmaceutical carrier may also be prepared, placed in an appropriate container, and labeled for treatment of an indicated condition, as is further detailed above.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.The references to the methods of treatment by therapy or surgery or in vivo diagnosis methods in this description, as part of the invention, are to be interpreted as references to compounds, pharmaceutical compositions and medicaments of the present invention for use in those methods.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated herein above and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

### MATERIALS AND METHODS

***Chemicals*** - Bt-cAMP (N6,2'-O-Dibutyryladenosine 3',5'-cyclic monophosphate, sodium salt, Sigma Catalog Number D0627, molecular weight 491.37) at a dose of 1 mM; and Theophylline (Theophylline anhydrous - 1,3-Dimethylxanthine, Sigma Catalog Number T1633, molecular weight 180.2) at a dose of 0.25-0.5 mM were used for astrocytes activation.

***Guiding Regenerative Gel (GRG)* -** Hyaluronic acid-based hydrogel containing the anti-oxidant sodium dismutase and a laminin peptide (KSIKVAVRSYIGSRCV, SEQ ID NO: 1), denoted herein as guiding regenerative gel (GRG), was generated as disclosed in International Patent Application Publication No. WO2009/022339. Briefly, e.g. a 0.4 % GRG was prepared from the three following components.
1. Hyaluronic Acid (HA): 1 Syringe of 2 ml gel of 1% Hyaluronic Acid (HA) in PBS (1:1 w/w) (Biotechnology-Ferring. Biotechnology General Israel Ltd. Israel) was added to a 50 ml tube.
2. Laminin peptide: Sixteen synthetic amino acids simulating laminin (SEQ ID NO: 1) containing the two cellular-biological active penta-peptides IKVAV-the epitope of laminin responsible for promoting neuronal outgrowth (SEQ ID NO: 2), and YIGSR the epitope of laminin responsible for promoting cell substrate adhesion (SEQ ID NO: 3) was obtained from LN, International Marketing Dept. of ChinaPeptide Co. Ltd., China. The laminin peptide was used in concentration of 50 µg solubilized in 1 ml of Phosphate buffered saline (PBS) and filtered through 0.45 micron filter paper. The filtered solution of 1 ml of LN peptide was added to the 50 ml tube containing the HA, all under sterile conditions.
3. Superoxide Dismutase (SOD): 20 µg SOD human recombinant (Merck Millipore Mercury, Israel) was solubilized in 1 ml of PBS.
Following, 1 ml of the sterilely filtered LM, 1 ml of sterile solution of SOD, 0.2 ml of hyaluronic acid and 2.8 ml PBS were mixed until receiving 5 ml of GRG in concentration of 0.4 %. The GRG was kept at 2-4 °C forming homogenous transparent gel.

***Acute complete spinal cord injury (SCI) model*** - Sprague-Dawley rats weighing approximately 250 gr each were operated and followed for up to 6 months. All surgical procedures were performed using a high magnification microscope. The spinal cord was exposed via a dorsal approach. The overlying muscles were retracted and the T7-T8 laminae removed, the spinal cord was completely transected using micro-scissors and a 2 mm segment of the cord was removed. Each animal was earmarked and randomly allocated to the various treatment groups, according to a randomization list generated before the initiation of the study:
1. Control group - rats with complete SCI with no further treatment.
2. Implantation of GRG in the transected area of the spinal cord, in direct contact with the margins of the two stumps.

The entire area of the lesion was covered with a thin biodegradable membrane (Lyodura, AESCULAP), composed of the biological co-polymer, attached by surgical adhesive (BioGlue, CryoLife) for fixation of the implants at the desired sites. Finally, the muscular and cutaneous planes were closed and sutured.

Post-operative animal care was performed to minimize discomfort and pain. The first two weeks after surgery are considered the most critical for the rats to survive; thus, analgesia was given during the first 5 days following the surgery, which includes antibiotics and pain medication. In addition, the rats were assisted in urination and defecation with the help of a veterinarian, twice a day. Animals were maintained in ventilated cages, containing sterile sawdust and sterile food. The paraplegic rats were kept solitary in cages, but were gathered in groups for 1 hour every day, in a large facility. The rats were monitored up to 6 months and at the termination of the experiments, the animals were sacrificed under general anesthesia.

***Statistical analysis*** - Chi-squared test was used to evaluate the statistical significance of survival rate between the treatment groups.

### EXAMPLE 1

### IN-VIVO EFFECTS OF GRG ON PREVENTION OF NEUROGENIC SHOCK AND MORTALITY FOLLOWING SPINAL CORD INJURY

To evaluate the effect of GRG on neurogenic shock and morbidity following SCI, the survival rate of rats has been evaluated (rats sacrificed for histology evaluation, during the first 7 weeks post-surgery, weren't taken into account). As shown in Figure 1, GRG had a protective effect against spinal shock: about half of the control rats died from spinal shock (due to the surgical SCI procedure) during the first 72 hours, while all the rats treated with GRG survived, and overall, the implantation of GRG decreased the mortality rate in the SCI treated rats at all time points tested.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### REFERENCES

### (Additional references are cited in text)

1. Piepmeyer JM, Lehmann KB and Lane JG. Cardiovascular instability following acute cervical spine trauma. Cent Nerv Syst Trauma, 1985; 2(3): 153-160.
2. Guly HR and Lecky FE. The incidence of neurogenic shock in patients with isolated Spinal cord injury in the emergency department. Resuscitation, 2008; 76(1): 57-62.

## Claims

1. A composition comprising a hyaluronic acid, a laminin polypeptide as set forth by SEQ ID NO: 1 and an antioxidant, for use in treating neurogenic shock following nerve injury in a subject demonstrating at least one symptom of neurogenic shock.

2. The composition for use according to claim 1, wherein said composition is implanted within 48 hours following said nerve injury.

3. The composition for use according to claim 1, wherein said composition is implanted within 24 hours following said nerve injury.

4. The composition for use according to claim 1, wherein said composition is implanted within 12 hours following said nerve injury.

5. The composition for use according to any one of claim 1-4, wherein said composition is implanted at or near said nerve injury of said subject.

6. The composition for use according to any one of claims 1-5, wherein said nerve injury comprises spinal cord injury (SCI).

7. The composition for use according to any one of claims 1-5, wherein said nerve injury comprises traumatic brain injuries (TBI) or traumatic optic neuropathy (TON).

8. The composition for use according to any one of claims 1-7, wherein said subject is demonstrating at least one symptom of said neurogenic shock selected from the group consisting of instantaneous hypotension, warm flushed skin, priapism, peripheral vasodilatation, inability to get tachycardic, bradycardia, diaphragmatic breathing, respiratory arrest and organ dysfunction.

9. The composition for use according to any one of claims 1-8, wherein said antioxidant is superoxide dismutase (SOD) or vitamin E.

10. The composition for use according to any one of claims 1-8, wherein said antioxidant is superoxide dismutase (SOD) comprising the amino acid sequence set forth by SEQ ID NO: 4.

11. The composition for use according to any one of claims 1-8, wherein said antioxidant is vitamin E.

12. The composition for use according to any one of claims 1-11, wherein said composition is formulated as a hydrogel.

13. The composition for use according to claim 12, wherein said laminin polypeptide is provided at a concentration range of about 20-100 µg/ml in said hydrogel.

14. The composition for use according to any one of claims 12-13, wherein said antioxidant is provided at a concentration range of about 5-40 µg/ml in said hydrogel.

15. The composition for use according to any one of claims 1-14, wherein said at least one symptom of neurogenic shock is selected from the group consisting of hypotension, bradycardia, and diaphragmatic breathing.

## Patentansprüche

1. Zusammensetzung, die eine Hyaluronsäure, ein Laminin-Polypeptid gemäß SEQ ID NO: 1 und ein Antioxidans umfasst, zur Verwendung bei der Behandlung eines neurogenen Schocks nach einer Nervenverletzung bei einem Patienten, der mindestens ein Symptom eines neurogenen Schocks aufweist.

2. Zusammensetzung zur Verwendung gemäß Anspruch 1, wobei die Zusammensetzung innerhalb von 48 Stunden nach der Nervenverletzung implantiert wird.

3. Zusammensetzung zur Verwendung nach Anspruch 1, wobei die Zusammensetzung innerhalb von 24 Stunden nach der Nervenverletzung implantiert wird.

4. Zusammensetzung zur Verwendung nach Anspruch 1, wobei die Zusammensetzung innerhalb von 12 Stunden nach der Nervenverletzung implantiert wird.

5. Zusammensetzung zur Verwendung nach einem der Ansprüche 1 bis 4, wobei die Zusammensetzung an oder in der Nähe der Nervenverletzung des Patienten implantiert wird.

6. Zusammensetzung zur Verwendung nach einem der Ansprüche 1 bis 5, wobei die Nervenverletzung eine Rückenmarksverletzung (SCI) umfasst.

7. Zusammensetzung zur Verwendung nach einem der Ansprüche 1-5, wobei die Nervenverletzung traumatische Hirnverletzungen (TBI) oder traumatische Optikusneuropathie (TON) umfasst.

8. Zusammensetzung zur Verwendung nach einem der Ansprüche 1 bis 7, wobei die Person mindestens ein Symptom des neurogenen Schocks zeigt, das aus der Gruppe ausgewählt ist, die aus augenblicklicher Hypotonie, warmer, geröteter Haut, Priapismus, peripherer Vasodilatation, Unfähigkeit, tachykard zu werden, Bradykardie, Zwerchfellatmung, Atemstillstand und Organdysfunktion besteht.

9. Zusammensetzung zur Verwendung nach einem der Ansprüche 1 bis 8, wobei das Antioxidans Superoxiddismutase (SOD) oder Vitamin Eist.

10. Zusammensetzung zur Verwendung nach einem der Ansprüche 1 bis 8, wobei das Antioxidans Superoxiddismutase (SOD) ist, das die durch SEQ ID NO: 4 dargestellte Aminosäuresequenz umfasst.

11. Zusammensetzung zur Verwendung nach einem der Ansprüche 1 bis 8, wobei das Antioxidans Vitamin Eist.

12. Zusammensetzung zur Verwendung nach einem der Ansprüche 1-11, wobei die Zusammensetzung als ein Hydrogel formuliert ist.

13. Zusammensetzung zur Verwendung nach Anspruch 12, wobei das Laminin-Polypeptid in einem Konzentrationsbereich von etwa 20-100 µg/ml in dem Hydrogel bereitgestellt wird.

14. Zusammensetzung zur Verwendung nach einem der Ansprüche 12 bis 13, wobei das Antioxidans in einem Konzentrationsbereich von etwa 5-40 µg/ml in dem Hydrogel bereitgestellt wird.

15. Zusammensetzung zur Verwendung nach einem der Ansprüche 1 bis 14, wobei das mindestens eine Symptom des neurogenen Schocks ausgewählt ist aus der Gruppe bestehend aus Hypotonie, Bradykardie und Zwerchfellatmung.

## Revendications

1. Composition comprenant un acide hyaluronique, un polypeptide laminine tel qu'exposé par SEQ ID NO: 1 et un antioxydant, pour une utilisation dans le traitement d'un choc neurogénique suivant une lésion nerveuse dans un sujet manifestant au moins un symptôme de choc neurogénique.

2. Composition pour une utilisation selon la revendication 1, dans laquelle ladite composition est implantée dans les 48 heures suivant ladite lésion nerveuse.

3. Composition pour une utilisation selon la revendication 1, dans laquelle ladite composition est implantée dans les 24 heures suivant ladite lésion nerveuse.

4. Composition pour une utilisation selon la revendication 1, dans laquelle ladite composition est implantée dans les 12 heures suivant ladite lésion nerveuse.

5. Composition pour une utilisation selon l'une quelconque des revendications 1-4, dans laquelle ladite composition est implantée au niveau ou près de ladite lésion nerveuse dudit sujet.

6. Composition pour une utilisation selon l'une quelconque des revendications 1-5, dans laquelle ladite lésion nerveuse comprend une lésion médullaire (SCI).

7. Composition pour une utilisation selon l'une quelconque des revendications 1-5, dans laquelle ladite lésion nerveuse comprend des traumatismes crâniens (TBI) ou une neuropathie optique traumatique (TON).

8. Composition pour une utilisation selon l'une quelconque des revendications 1-7, dans laquelle ledit sujet manifeste au moins un symptôme dudit choc neurogénique sélectionné dans le groupe consistant en hypotension instantanée, rougissement, priapisme, vasodilatation périphérique, incapacité à la tachycardie, bradycardie, respiration diaphragmatique, arrêt respiratoire et dysfonctionnement organique.

9. Composition pour une utilisation selon l'une quelconque des revendications 1-8, dans laquelle ledit antioxydant est une superoxyde dismutase (SOD) ou une vitamine E.

10. Composition pour une utilisation selon l'une quelconque des revendications 1-8, dans laquelle ledit antioxydant est une superoxyde dismutase (SOD) comprenant la séquence d'acide aminé exposée par SEQ ID NO: 4.

11. Composition pour une utilisation selon l'une quelconque des revendications 1-8, dans laquelle ledit antioxydant est une vitamine E.

12. Composition pour une utilisation selon l'une quelconque des revendications 1-11, dans laquelle ladite composition est formulée comme un hydrogel.

13. Composition pour une utilisation selon la revendication 12, dans laquelle ledit polypeptide laminine est fourni dans une gamme de concentration d'environ 20-100 µg/ml dans ledit hydrogel.

14. Composition pour une utilisation selon l'une quelconque des revendications 12-13, dans laquelle ledit antioxydant est fourni dans une gamme de concentration d'environ 5-40 µg/ml dans ledit hydrogel.

15. Composition pour une utilisation selon l'une quelconque des revendications 1-14, dans laquelle ledit au moins un symptôme de choc neurogénique est sélectionné dans le groupe consistant en hypotension, bradycardie et respiration diaphragmatique.
